(19) Europäisches Patentamt
European Patent Office
Office européen des brevets

(11) **EP 2 722 064 B1**

(12) **EUROPÄISCHE PATENTSCHRIFT**

(45) Veröffentlichungstag und Bekanntmachung des Hinweises auf die Patenterteilung:
**20.04.2016 Patentblatt 2016/16**

(51) Int Cl.:
***A61M 1/14*** *(2006.01)*

(21) Anmeldenummer: **13187980.1**

(22) Anmeldetag: **09.10.2013**

(54) **Dialyseoptimierungsverfahren**

Dialysis optimisation method

Procédé d'optimisation de dialyse

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **16.10.2012 DE 102012109858**

(43) Veröffentlichungstag der Anmeldung:
**23.04.2014 Patentblatt 2014/17**

(73) Patentinhaber: **B. Braun Avitum AG**
**34212 Melsungen (DE)**

(72) Erfinder:
• **Meibaum, Jörn**
**34225 Baunatal (DE)**

• **Strohhöfer, Christof**
**34123 Kassel (DE)**

(74) Vertreter: **Winter, Brandl, Fürniss, Hübner, Röss, Kaiser, Polte - Partnerschaft mbB Patent- und Rechtsanwaltskanzlei Bavariaring 10 80336 München (DE)**

(56) Entgegenhaltungen:
**EP-A1- 0 928 614    EP-A1- 2 163 271 US-A- 5 849 179**

**Beschreibung**

Die vorliegende Erfindung betrifft ein Verfahren sowie eine Vorrichtung zur Bestimmung der Effektivität einer aktuell durchgeführten Nierenersatzbehandlung bzw. der Behandlungseinrichtung.

Hintergrund der Erfindung

Patienten, die an einer Niereninsuffizienz leiden, müssen sich urämische Toxine, welche sich über die Zeit im Patientenkörper anlagern, mittels einer extrakoporalen Nierenersatztherapie, nachfolgend als Dialysebehandlung bezeichnet, entfernen lassen, sofern, bzw. solange keine Nierentransplantation in Aussicht gestellt ist. Durch eine solche Dialysebehandlung wird dem Patienten Blut entnommen, über eine extrakorporale Reinigungsvorrichtung - den Dialysator - geführt und anschließend wieder in den Patientenkörper zurückgegeben. Im Dialysator kommt dann das zu reinigende Blut mit einer Waschlösung - der frischen Dialysierflüssigkeit - über eine semipermeable Membran in Kontakt, was zu einem Stoffaustausch führt. Das nun mit urämischen Toxinen angereicherte Dialysat wird anschließend ausgespült und verworfen.

Stand der Technik

Für die Bewertung einer Dialysetherapie ist die Effektivität der vorstehend beschriebenen Dialysebehandlung bzw. der verwendeten Dialysemaschine von herausragender Bedeutung. In der Vergangenheit wurden daher unterschiedliche Verfahren entwickelt und eingesetzt, welche eine Beurteilung der Therapie bzw. des Maschinenzustands jeweils nach deren Abschluss erlauben. In der jüngeren Vergangenheit liegt der Fokus jedoch darauf, die Effektivität einer Dialysetherapie bereits während der Behandlung selber zu ermitteln, um eventuell korrigierend manuell eingreifen zu können.

Um eine effektive Dialysetherapie zu gewährleisten, wurde daher das sogenannte
K*t/V (Harnstoff) - Modell entwickelt mit
K = Reinigungsleistung des Dialysators (ml/min),
t = Dialysezeit (min) und
V = Harnstoffverteilungsvolumen im Patienten (ml).

Darüber hinaus wurde auch die Harnstoffreduktionsrate (URR, in %) als geeignetes Mittel zur Bestimmung der Harnstoffreduktion im menschlichen Körper eingeführt.

Die NCDS (national cooperative dialysis study) sowie die HEMO (Hämodialyse)-Studie belegen nach der Untersuchung einer Vielzahl von Dialysepatienten, dass die Mortalität und Morbidität in ESRD (end-stage-renal-disase)-Patienten sehr stark mit dem genannten K*t/V -Wert (nämlich der Dialysedosis) korreliert. Daten aus den genannten Studien führen beispielsweise dazu, dass in den Richtlinien zur Durchführung einer geeigneten Dialysetherapie eine Behandlung erst dann als effektiv gilt, wenn ein Wert K*t/V >=1,2 erreicht wird.

Es sei an dieser Stelle darauf hingewiesen, dass es sich bei der Beurteilung der Dialysedosis mittels eines K*t/V - Werts um eine relative Einschätzung, ähnlich der vorstehend genannten URR handelt. Eine absolute Aussage über die tatsächlich entfernte Stoffmenge erlaubt dieses Verfahren jedoch nicht.

In der K*t/V- Bestimmung sind die Bestimmung der Reinigungsleistung K und des Harnstoffverteilungsvolumens V, bedingt durch die Harnstoffkinetik, schwierig. V kann beispielsweise durch eine Bioimpedanzmessung, empirische Abschätzungen oder durch die Berechnung mittels des bekannten Harnstoffkinetikmodells (UKM) mehr oder weniger gut bestimmt werden. K kann durch die Bestimmung von Harnstoff im Blut des Patienten vor und nach einer Dialysetherapie oder durch die Bestimmung der Änderung der Leitfähigkeit am Dialysatoreingang beziehungsweise am Dialysatorausgang des Dialysatkreislaufs ermittelt werden.

Die Entnahme von Blutproben ist dabei die Referenzmethode. Nach der Probenentnahme sowie der Verrechnung mit dem UKM oder der an sich bekannten Daugirdas - Formel erhält man einen K*t/V - Wert, der auf einem Kompartiment als Berechnungsgrundlage basiert (spKTV). Es existiert ferner eine durch Daugirdas weiterentwickelte Formel, welche nunmehr einen äquilibrierten K*t/V (eKTV)- Wert liefert, der die Harnstoffgeneration sowie den Rebound mit berücksichtigt. Dieses bekannte Verfahren hat jedoch einen entscheidenden Nachteil:

Die Bewertung der Dialysedosis spK*t/V bzw. eK*t/V ist wiederum erst nach Abschluss der Dialysebehandlung möglich, da nach wie vor die Harnstoffblutwerte vom Patienten benötigt werden.

Leitfähigkeitsmessverfahren beruhen schließlich auf der Annahme, dass die Natrium-Reinigungsleistung des Dialysators fast identisch mit der von Harnstoff ist. Das Verhältnis von

"Natriumleitfähigkeit" zu "Natriumkonzentration"

ist jedoch darüber hinaus von der Temperatur abhängig. Da die Temperaturänderung durch die Temperierung des Dialysats jedoch nur gering ist, wird diese im betrachteten Temperaturbereich (z.B. 37°C +/- 2°C) als konstant angenommen. Daher ist es möglich, die Reinigungsleistung von Harnstoff durch die Messung der Reinigungsleistung von Natrium zu bestimmen. Die Vorteile dieses Verfahrens liegen darin, dass sie relativ leicht zu implementieren und dabei auch kostengünstig ist. Sie bietet K*t/V - Messungen während der Dialysetherapie und erlaubt so korrigierende Eingriffe seitens einer Bedienperson bereits vor Abschluss der Behandlungen.

Jedoch haben auch Leitfähigkeitsmessungen gemäß vorstehender Beschreibung Nachteile:

Zum Einen führt das Verfahren dazu, dass dem Patienten ungewollt/notgedrungen Natrium verabreicht wird, zum Anderen wurden in der Praxis Messintervalle von minimal 20min erreicht, was effektiv ca. 12 Messungen innerhalb einer durchschnittlichen Dialysetherapie von 4 Std. bedeutet. Abschließend sind Leitfähigkeitsmessverfahren fehleranfällig, da die Messung von anderen Substanzen mit ionischen Eigenschaften beeinflusst wird, welche die Gesamtleitfähigkeit verändern/beeinflussen und somit die Berechnung der Natriumkonzentration erschweren/verfälschen.

Ein weiteres Verfahren zur Bestimmung der Dialyseeffektivität ist die direkte Messung der urämischen Substanzen (z.B. Harnstoff) im verbrauchten Dialysat.

Dazu gibt es zwei Möglichkeiten, von denen beide die direkte Bestimmung der Reinigungsleistung bzw. des Harnstoffverteilungsvolumens umgehen:

- Eine erste Möglichkeit besteht darin, die Änderung der Harnstoffkonzentration im Blut wie im verbrauchten Dialysat als linear anzunehmen. Daher ist die Steigung der Kurve, welche man erhält, nachdem man den natürlichen Logarithmus der aufgenommenen Konzentrationen bestimmt hat, im Dialysat wie im Blut identisch. Das Problem dieses Verfahrens wird jedoch deutlich, wenn während der Therapie eine Reduktion der Reinigungsleistung z.B. durch eine sogenannte Rezirkulation oder durch Verblockung des Dialysators auftritt. Dies führt nämlich zu einer erhöhten Steigung und somit zu einem scheinbar verbesserten K*t/V-Wert, obwohl dies gar nicht der Fall ist.

- Eine zweite Möglichkeit ist insbesondere im Stand der Technik gemäß der EP 0 986 410 beschrieben. In dieser Druckschrift wird eine gute Korrelation der "whole body clearance (wbK*t/V) und dem Daugirdas - K*t/V gefunden. Dieser Ansatz nimmt die Reinigungsleistung des Dialysators als konstant innerhalb bestimmter Zeitintervalle an und berechnet so den K*t/V - Wert quasi "rückwärts". Obwohl diese zweite Möglichkeit per se sicherer ist als die vorstehend geschilderte erste Möglichkeit, basiert sie dennoch auf der Annahme von konstanten Bedingungen innerhalb bestimmter Zeitintervalle und kann daher den wbK*t/V - Wert zu Beginn der Therapie nicht berechnen.

Die Verfahren zur Bestimmung von Konzentrationen urämischer Toxine betreffen Harnstoffsensoren und die UV-Spektrophotometrie. Die technischen Einschränkungen bei der Verwendung von Harnstoffsensoren liegen jedoch in ihrer Stabilität und Wiederverwertbarkeit.

Des Weiteren wurde gezeigt, dass mit der UV-Spektroskopie eine sehr gute Korrelation zu der Konzentration urämischer Substanzen (z.B. Harnsäure, Harnstoff, etc.) im verbrauchten Dialysat hergestellt werden kann. Da mit diesem Verfahren ein Maß für die Konzentration urämischer Toxine im verbrauchten Dialysat vorliegt, ist es möglich, die relative Änderung urämischer Toxine im Sinn eines K*t/V - Werts darzustellen. Aber auch dieses Verfahren hat einen Nachteil:

Verminderungen der Reinigungsleistung des Dialysators z.B. durch Verblockung werden mit diesem Verfahren nicht erfasst. Obwohl die Reinigungsleistung dramatisch reduziert würde, erhöht sich die Anzeige des K*t/V - Werts in diesem Fall. Darüber hinaus können physiologische Änderungen im Patienten während der Dialysetherapie fehl - interpretiert werden.

Kommt es z.B. durch Vasokonstriktion, Wasseraufnahme oder andere metabolische Besonderheiten zu einer plötzlichen / abrupten Änderung der Konzentration urämischer Toxine im Blut des Patienten, so werden diese Änderungen durch die relative Betrachtungsweise des K*t/V - Werts ggf. fehl - interpretiert (zu gut oder zu schlecht) und geben daher nicht den tatsächlichen Patientenstatus wider. Derzeit lässt sich die Konzentration einer Substanz im Blut des Patienten daher fast ausschließlich nur durch die chemische Analyse des Bluts selbst bestimmen.

Die EP 2 005 982 B1 beschreibt schließlich ein Verfahren, bei dem durch eine geeignete Fluidführung in der Dialysemaschine die Konzentration urämischer Toxine im Dialysat die gleiche Höhe erreicht, wie im Blut des Patienten. Dies wird erreicht durch eine zeitlich begrenzte Zirkulation des Dialysats durch den Dialysator während das Blut weiterhin therapiegemäß zwischen Patient und Dialysator ausgetauscht wird. Dabei kommt es zu einer Sättigung des Dialysats mit urämischen Toxinen. Die Konzentration dieser Toxine, bzw. der Absorptionskoeffizient bei der analysierten Wellenlänge, der durch dialysatorgängige Toxine im Blut verursacht wird, lässt sich damit im Dialysat direkt messen.

Wie vorstehend bereits ausgeführt wurde, weisen Verfahren, welche die Dialysedosis in Echtzeit bestimmten, Schwächen im Bereich der Interpretation physiologischer Änderungen im Patienten bzw. Änderungen der Dialysatoreigenschaften (Reinigungsleistung) in Bezug auf die Berechnung des K*t/V -Werts auf. Da dieser Wert immer die relative Änderung beschreibt, kommt es hier zwangsläufig zu Fehl - Interpretationen. Die Dialysedosis im Sinn eines K*t/V - Werts wird in diesem Fall je nach Situation entweder über- oder unterschätzt.

Ein anderes Problem tritt bei Verfahren auf, welche durch Blutprobenentnahme den K*t/V - Wert auf Basis der Harnstoffkonzentration ermitteln. Auch sie bestimmen lediglich die relative Änderung der Harnstoffkonzentration vor und nach einer Dialysetherapie und ermitteln den K*t/V - Wert auf Basis von Daugirdas. Dabei wird je nach Verfahren ein Ein - Kompartiment oder ein Doppel - Kompartiment als Modellfunktion angenommen und angenähert. Physiologische Änderungen im Patienten, beispielsweise durch Nahrungsaufnahme oder Änderungen des physiologischen Zustands werden nur bedingt berücksichtigt.

Nachteil des beschriebenen Verfahrens zur Sättigung des Dialysats mit der blutseitigen Konzentration urämischer Toxine ist seine aufwändige Implementierbarkeit in einer Dialysemaschine. Hierzu ist der Einsatz von zusätzlichen Schlauchverbindungen und entsprechenden Ventilen sowie Steuereinheiten notwendig, um in einen Zirkulationsbetrieb umzuschalten, der die Sättigung des Dialysats erst ermöglicht. Zudem muss jener Sensor, mit dem die Konzentration bestimmt wird, an dem entsprechenden Zirkulationskreislauf liegen, was maschinenseitig eine aufwändige Realisierung bedeutet, da zugleich das Volumen des Zirkulationskreislaufs möglichst klein gehalten werden muss. Entsprechend müssen hier auch Kompromisse bei der Gesamtmesszeit eingegangen werden.

US5849179 A offenbart eine weitere Dialysemaschine gemäß dem Stand der Technik.

Angesichts der vorstehend geschilderten Problematik besteht die Aufgabe der vorliegenden Erfindung darin, eine Messmethodik zu entwickeln, welche es ermöglicht, die Konzentration urämischer Toxine im Blut dialysepflichtiger Patienten dialyseseitig zu bestimmen. Ein Ziel hierbei ist es, dass dieses Messverfahren ohne den direkten Kontakt mit dem Patienten auskommt und somit nicht als interventionell im diesem Sinn betrachtet werden kann. Das erfindungsgemäße Verfahren soll ferner die auf der Dialyseseite (in der Dialysemaschine angewandten) bekannten Verfahren ausnutzen, um die blutseitige Konzentration urämischer Toxine zu messen. Vorzugsweise soll dies während eines Dialysetherapie durchgeführt werden, ohne diese unterbrechen zu müssen.

Diese Aufgabe durch eine Dialysemaschine mit den Merkmalen des Anspruchs 1 gelöst. Vorteilhafte Weiterbildungen sind dabei Gegenstand der Unteransprüche.

Gemäß einem Aspekt der vorliegenden Erfindung wird demnach ein Verfahren zur Bestimmung der Effektivität einer aktuell durchgeführten Nierenersatzbehandlung auf der Dialyseseite unter Verwendung einer Dialysemaschine vorgestellt, die in einem ersten Schritt in einem Hämodialyse- oder Hämodiaflitrationsprozess betrieben wird und in einem zweiten Schritt in einen Hämofiltrationsprozess sequenziell umgeschaltet wird bzw. in einen sequentiellen Modus, in dem lediglich ein Fluss vom Blutkompartment (Flüssigkeitsentzug des Patienten) über die semipermeable Membran zum Dialysierflüssigkeitskompartment des Dialysators erzeugt wird, umgeschaltet wird, wonach gemäß einem dritten Schritt ein dialyseseitig einem Dialysator nachgeschalteter Sensor zur Bestimmung oder Messung der Konzentration zumindest von urämischen Toxinen im angesättigten Dialysat (Ultrafiltrat) entsprechende Messsignale an eine Berechnungs- oder Bestimmungseinheit ausgibt, welche repräsentativ für die aktuelle Konzentration zumindest von urämischen Toxinen im Blut des Patienten sind.

Unter Hämofiltration wird allgemein ein Verfahren verstanden, bei dem die Konzentrationen wasserlöslicher Substanzen im Patientenblut und ein Überschuss an Patientenflüssigkeit korrigiert werden durch unidirektionalen konvektiven Transport mittels Ultrafiltration über die semipermeable Membran, die Blut von Dialysierflüssigkeit trennt und Ultrafiltrat zeitgleich durch eine annähernd isoosmolare Substitutionsflüssigkeit mit einer entsprechenden Rate ersetzt wird, sodass die Differenz der Raten zwischen der Ultrafiltration und Zugabe von Substitutionsflüssigkeit den Nettoflüssigkeitsentzug ergibt.

Vorzugsweise werden der Umschaltschritt sowie der nachfolgende Messschritt mindestens zu Beginn, und vorzugsweise auch während und am Ende einer Dialysebehandlung ausgeführt. Des Weiteren soll nach Beendigung des Messschritts die Dialysemaschine erneut in den Hämodialyse- oder Hämodiaflitrationsprozess ohne die Notwendigkeit eines die Dialyse zeitverlängernden Zirkulationsprozesses zurückgeschaltet werden.

Die vorliegende Erfindung betrifft eine Dialysemaschine mit einer Messeinrichtung zur Messung und/oder Bestimmung der Konzentration zumindest von urämischen Toxinen in Patientenblut. Diese erfindungsgemäße Dialysemaschine hat hierbei die folgenden technischen Merkmale:

Einen Dialysator, der auf seiner Eingangsseite von wenigstens einer Versorgungspumpe mit einer frischen Dialysierflüssigkeit anströmbar ist und wenigstens eine Entsorgungspumpe, welche an die Ausgangsseite des Dialysators angeschlossen ist, um das zumindest mit urämischen Toxinen kontaminierte Dialysat aus dem Dialysator zu fördern.

Erfindungsgemäß ist eine Ventileinrichtung ggf. bestehend aus mehreren Schließventilen vorgesehen, welche in einem Hämodialyse- oder Hämodiaflitrationsmodus der Maschine eine Fluidverbindung der wenigstens einen Versorgungspumpe zum Dialysator zulässt und in einem Hämofiltrationsmodus der Maschine diese Fluidverbindung unterbricht und stattdessen einen Umgehungskanal zum Dialysator öffnet und wenigstens eine Sensoreinrichtung, welche dem Dialysator nachgeschaltet und dafür ausgebildet ist, im Hämofiltrationsmodus der Maschine, bzw. in einem sequentiellen Modus, in dem lediglich ein Fluss vom Blutkompartment (Flüssigkeitsentzug der Patienten) über die semipermeable Membran zum Dialysierflüssigkeitskompartment des Dialysators erzeugt wird, Messsignale an eine Berechnungs- und/oder Bestimmungseinheit zu senden, welche die Konzentration zumindest von urämischen Toxinen in dem Flüssigkeitsgemisch aus Ultrafiltrat aus dem Blut und der Dialysierflüssigkeit im Dialysatorkompartment des Dialysators (nachfolgend der Einfachheit als "Ultrafiltrat" bezeichnet) angeben sowie repräsentativ für die Toxinen - Konzentration im Patientenblut sind.

An dieser Stelle sei darauf hingewiesen, dass in der Praxis im Ultrafiltrat erst dann gemessen werden kann, wenn das Dialysierflüssigkeitskompartment des Dialysators mit Ultrafiltrat gefüllt ist. Dieses entsteht aber erst nach einigen Minuten. Jedoch ist der Vorgang der Anreicherung mit Toxinen an der Messvorrichtung in der Regel wesentlich schneller. Dieses ist mit dem diffusiven Stofftransport der urämischen Toxine zu begründen, der aufgrund des Konzentrationsgefälles zwischen Blut und Dialysierflüssigkeit wesentlich schneller ist als der konvektive Stofftransport.

Hämodialyse, Hämodiafiltration und Hämofiltration sind bekannter Maßen Behandlungsverfahren (Behandlungsmodi), bei denen das Blut mittels Diffusion und Konvektion behandelt wird. Bei der Hämodialyse wird im Wesentlichen das Blut mittels Diffusion behandelt, während bei der Hämofiltration das Blut mittels Konvektion behandelt wird. Die Hämodiafiltration kombiniert beides, nämlich Diffusion durch den Dialysierflüssigkeitsfluss im Dialysierflüssigkeitskompartment des Dialysators und den Substitutionsfluss in den extrakorporealen Kreislauf. Im Hämofiltrationsmodus wird der Zulauf zum Dialysierflüssigkeitskompartment des Dialysators unterbrochen. Die Blutbehandlung findet dann durch den Substitutionsfluss in den extrakorporealen Kreislauf statt.

Im vorstehend erwähnten sequentiellen Modus wird lediglich ein Fluss vom Blutkompartment über die semipermeable Membran zum Dialysierflüssigkeitskompartment des Dialysators erzeugt, während im Hämofiltrationsmodus Substitutionsflüssigkeit in den extrakorporealen Kreislauf gepumpt wird, sodass über die semipermeable Membran die Summe aus Flüssigkeitsentzug des Patienten und dem Substitutionsfluss besteht.

Der Zusammenhang der Flüsse ergibt sich dabei wie folgt:

Der Fluss durch die Sensoreinrichtung hängt vom Behandlungsmodus ab. Bei Hämodialyse ist dies die Summe aus Dialysierflüssigkeitsfluss und Ultrafiltrationsfluss. Bei Hämofiltration ist dies die Summe aus Substitutionsfluss und Nettoflüssigkeitsentzug. Bei Hämodiafiltration ist dies die Summe aus Dialysierflüssigkeitsfluss plus Substitutionsfluss und Nettoflüssigkeitsentzug. Schließlich ist die bei sequentieller Behandlungsart der Ultrafiltrationsfluss oder Nettoflüssigkeitsentzug.

Vorzugsweise kann es vorgesehen sein, dass die Sensoreinrichtung an eine Verbindungsleitung zwischen dem Dialysator und der Entsorgungspumpe stromauf zu dem Umgehungskanal angeschlossen ist. Alternativ oder zusätzlich kann die Sensoreinrichtung der Entsorgungspumpe nachgeordnet sein.

Mit den vorstehenden Merkmalen lassen sich gegenüber dem bekannten Stand der Technik die nachfolgenden Vorteile erzielen:

- Einfache Implementierung in allen bestehenden Dialysemaschinen, da die Bereitstellung der blutseitigen Konzentration erfindungsgemäß auf der Dialysatseite durch in allen Maschinen bereits vorhandene Komponenten, nämlich einer ggf. zusätzlichen Ultrafiltrationspumpe, erfolgt.

- Die Abfuhr urämischer Toxine wird nie vollständig unterbrochen, wie es in einer Zirkulationsphase der Fall ist. Stattdessen wird zwischen einer Hämodialyse- oder Hämodiafiltrationsphase und einer Hämofiltrationsphase und einer sequenziellen Phase geschaltet (sequenziell geschaltet), d.h. es werden nur etablierte Blutreinigungsverfahren verwendet, sodass sich hierbei keine Verzögerung der Dialysetherapie ergibt.

- Die blutseitige Konzentration wird bei der bekannten Zirkulationsmethode erst nach einer gewissen Einschwingzeit erreicht, die durch die Geschwindigkeit der Sättigung bestimmt ist. Bei dem erfindungsgemäß vorgeschlagenen Verfahren liegt die blutseitige Konzentration sofort bei Übergang zur sequentiellen Phase (sequentieller Modus) in der Flüssigkeit im Dialysierflüssigkeitskompartment des Dialysators (Filtrationsphase im Filtrat) vor. Dies erlaubt eine zügigere Bestimmung der Konzentration in der Flüssigkeit im Dialysierflüssigkeitskompartment am Ausgang des Dialysators (im Filtrat auf der Dialysatseite).

- Die Anordnung des Sensors zur Konzentrationsmessung ist im vorgeschlagenen Fall weniger kritisch. Dieser kann prinzipiell beliebig im Abfluss des Dialysats/Ultrafiltrats positioniert werden (aus Genauigkeitserwägungen ist eine Position vor der Entsorgungspumpe jedoch vorzuziehen). Die Realisierung dieses Verfahrens bedingt also eine geringere Komplexität beim Zusammenbau der Maschine.

In anderen Worten ausgedrückt betrifft der Gegenstand der aktuellen Entwicklung demnach die Realisierung eines Verfahrens zur Beurteilung der absoluten Konzentration urämischer Toxine im Blut von Dialysepatienten auf der Dialyseseite und nicht auf der Blut-/Patientenseite. Dies wird vorzugsweise durch die Messung von optischen Eigenschaften im verbrauchten Dialysat/Ultrafiltrat während einer sequentiellen Ultrafiltrationsphase umgesetzt. Darüber hinaus ist mittels einer Erweiterung dieses Verfahrens die Bestimmung der Reinigungsleistung (Clearance) des Dialysators möglich.

Wie bereits geschildert, ist die Bewertung einer Therapie mittels der relativen Dialysedosis K*t/V mit einigen Schwächen verbunden. Durch die Messung eines Maßes zur Bestimmung der absoluten Konzentration im Blut zu Beginn der Dialysetherapie gemäß der vorliegenden Erfindung kann jedoch die tatsächlich entfernte Menge an urämischen Toxinen im verbrauchten Dialysat/Ultrafiltrat als Grundlage zur Bewertung einer Dialysetherapie herangezogen werden. Man spricht hier von der absoluten Menge entfernter Stoffe über eine Dialysetherapie. Dieses Verfahren verfügt gleich über mehrere Vorteile:

Zum Einen werden Änderungen der Reinigungsleistung durch Verblockung des Dialysators nicht fehl - interpretiert, da nur die tatsächlich entfernte Menge an urämischen Toxinen, welche den Dialysator passieren, gemessen wird. Dasselbe gilt auch für physiologische Änderungen im Patienten. Kommt es nämlich zu einem Anstieg oder Abfall urämischer Toxine im Blut und somit zwangsläufig auch im verbrauchten Dialysat/Ultrafiltrat, so wird auch hier lediglich die tatsächlich entfernte Stoffmenge ermittelt, unabhängig zur bereits entfernten Stoffmenge (nicht relativ).

Mit dem gleichen Verfahren, welches im Folgenden genauer beschrieben wird, ist es zum Anderen möglich, die Reinigungsleistung des Dialysators zu jedem beliebigen Zeitpunkt, aber vor allem zu Beginn einer Dialysetherapie zu quantifizieren. Dies ist auch im Hinblick auf die Bewertung eines Dialysators vor Beginn der eigentlichen Behandlung, vor allem bei der Wiederverwendung gebrauchter Dialysatoren, sinnvoll. Weiterhin kann die Anreicherung von urämischen Toxinen im Patienten über einen großen Zeitraum hinweg (mehrere Wochen / Monate) beobachtet und so der Grad des Fortschreitens der Erkrankung beobachtet werden. Als Messeinrichtungen können jede Art von Sensoren oder Verfahren verwendet werden, die eine Bestimmung absoluter Konzentrationen ermöglichen. Als Beispiel sind hier sowohl Harnstoffsensoren als auch UV-Messeinrichtungen genannt. Die präferierte Methode in dieser Erfindungsmeldung ist die Nutzung von UV-Spektrometrie.

Das im Folgenden beschriebene Messverfahren kann generell zu jedem Zeitpunkt einer Dialysetherapie durchgeführt werden. Setzt man es zur Charakterisierung von sogenanntem "Clotting" bzw. "Blocking" des Dialysators ein, ist es sinnvoll, dass es regelmäßig während der Dialysetherapie durchgeführt wird. Jedoch reicht es in den meisten Fällen aus, das Messverfahren einmal zu Beginn der Behandlung anzuwenden.

Zu Beginn einer Dialysetherapie sind die Dialysatoreigenschaften sowie die Patientensituation (Shunt, Ernährungszustand etc.) erfahrungsgemäß am stabilsten, wenn man die gesamte Dauer einer Dialysetherapie betrachtet. Diesen Umstand kann man nutzen, um mit Hilfe einer erfindungsgemäßen sequentiellen Phase beispielsweise die Absorbanz als Maß für die Konzentration urämischer Toxine absolut zu bestimmen.

Durch die Aktivierung der sequentiellen Phase wird der Dialysatfluss über den Dialysator gestoppt (umgeleitet), so dass lediglich das Ultrafiltrat (gemäß vorstehender Definition) die Messeinrichtung (Sensor) zur Bestimmung absoluter Konzentrationen, welcher im Dialysatabfluss angebracht ist, erreicht. Aufgrund der Tatsache, dass der Ultrafiltration im Wesentlichen die Konvektion als Stofftransport über die semipermeable Membran beiträgt, ändert sich auch die Konzentration urämischer Toxine nicht. D.h., dass das entzogene Ultrafiltrat (eigentlich Flüssigkeitsgemisch gemäß vorstehender Definition) im Wesentlichen die Menge an urämischen Toxinen enthält, welche auch im Blut vorhanden ist. Da nach Lambert-Beer (1) die Absorbanz wässriger Flüssigkeiten als Maß für die Konzentration optisch aktiver Substanzen geeignet ist, kann somit die absolute, d.h. blutseitige, Konzentration urämischer Toxine dialyseseitig (über/anhand des Ultrafiltrats) bestimmt werden. Nach Abschluss dieser Messung kann die sequentielle Phase sofort wieder deaktiviert werden. Eine Unterbrechung der Dialysetherapie wird somit umgangen, da auch die sequentielle Phase zur Reinigung des Blutes geeignet ist.

Für das Beispiel einer UV-Messeinrichtung gilt:

$$(1)$$

$$A_{UF} = \log\left(\frac{I_0}{I_t}\right) \text{ bzw. } A = \varepsilon \cdot c \cdot l \Rightarrow A \sim c, \text{ falls } \varepsilon, l = \text{konst.}$$

$I_0$ entspricht dabei der Intensität der UV-Messeinrichtung mit einer Flüssigkeit, in der sich keinerlei urämische Toxine befinden. $I_t$ hingegen bezieht sich auf die gemessene Intensität zu einem beliebigen Zeitpunkt t. $\varepsilon$ bezeichnet den Extinktionskoeffizient, welcher für bekannte Substanzen ebenfalls bekannt ist. Abschließend stellt $I$ die optische Weglänge des Lichtes der UV-Strahlung durch die zu untersuchende Lösung dar. Die Formel weist daraufhin, dass zunächst die optischen Eigenschaften von unverbrauchtem Dialysat, d.h. ohne urämische Toxine, bekannt sein muss. Diese Messung zur Bestimmung von $I_0$ kann bereits während des Vorbereitens der Dialysemaschine stattfinden. Abschließend stellt $A_{UF}$ die Absorbanz im Ultrafiltrat als Maß (stellvertretend) für die Konzentration urämischer Toxine im Blut des Patienten dar.

Die Gleichung (1) ermöglicht es zunächst, den Absorptionskoeffizienten $\alpha = \varepsilon * c$ mittels Division durch den optischen Pfad in einer Absorptionsmessküvette zu berechnen. Der Absorptionskoeffizient beschreibt die spezifische Absorption bei der Messwellenlänge, die die urämischen Toxine im Blut haben. Ist darüber hinaus $\varepsilon$ bekannt, z.B. weil bei der verwendeten Wellenlänge nur ein bestimmter Stoff absorbiert wird, oder weil der spezifische Anteil eines Stoffes am Absorptionskoeffizienten aufgrund gängiger Messmethoden aus einem Mischsignal isoliert werden kann, so ergibt sich die Konzentration des Stoffes als zusätzliche Information aus dem Absorptionskoeffizienten.

Mit dem Maß für die Startkonzentration urämischer Toxine im Blut kann nun über das kontinuierliche Messen verbrauchten Dialysates/Ultrafiltrats die tatsächlich entfernte Stoffmenge über die Dialysetherapie hinweg quantifiziert werden. Am Ende der

Therapie steht neben der K*t/V - Berechnung, welche durch diese Messung nicht beeinflusst wird, eine zusätzliche Information zur Verfügung, welche von Änderungen der Dialysatoreigenschaften bzw. des Patientenstatus nicht verfälscht wird. Im Gegenteil dazu können solche Änderungen sogar aufgezeichnet werden und bei einer Darstellung auf dem Bildschirm u.U. auf Missstände/Änderungen hinweisen bzw. auf eine notwendige Interaktion der Pflegekraft aufmerksam machen.

Um eine möglichst reibungslose und schnelle Messwertaufnahme zu ermöglichen, sollte sich die Messeinrichtung möglichst nah am Dialysatabfluss des Dialysators (Ausgangsseite) befinden. Allerdings ist prinzipiell jede Position nach dem Dialysatorausgang bis zum Abfluss denkbar. Vorzuziehen ist dabei eine Position im Abfluss vor einer Bilanzierungseinrichtung der Dialysemaschine.

Denkbar ist auch, dass die Durchführung der sequentiellen Phase bereits abgebrochen wird, bevor das Ultrafiltrat den Sensor erreicht. Zur Durchführung des beschriebenen Verfahrens ist ein für die Messzwecke ausreichender Puls ausreichend. Ist nämlich die Position des Sensors bekannt, so kann über Kenntnis des internen Dialysatvolumens in den Schlauchsystemen sowie des Dialysatflusses nach Abbruch der sequentiellen Ultrafiltrationsphase die Zeit abgeschätzt werden, bis der Puls den Sensor erreichen wird. Hierzu wird ein Puls benötigt, welcher schätzungsweise im Bereich 1 bis 250ml liegt. Je nach Ultrafiltrationsrate während der sequentiellen Phase wird hierzu schätzungsweise eine Zeit von wenigen Sekunden bis 5 Minuten benötigt.

Alternativ dazu kann der Ultrafiltrationsfluss auch dazu verwendet werden, die bereits im Dialysator angesättigte Waschlösung innerhalb einer sequentiellen Phase zur Messeinrichtung zu befördern. Geht man von der Tatsache aus, dass diffusive Stofftransporte weit schneller vonstatten gehen als ein konvektiver Stofftransport, dann würde der Ultrafiltrationsfluss neben dem konvektiven Anteil auch den Anteil der Dialysatlösung zur Messeinrichtung befördern, welcher mittels Diffusion angereichert wurde. Beide Effekte bedingen sich gegenseitig, führen jedoch zum gleichen Ergebnis:

Die durch den Ultrafiltrationsfluss zur Messeinrichtung beförderte Messlösung besitzt die gleiche Konzentration an urämischen Toxinen wie das Blut.

Der Vorteil dieser Art der Umsetzung liegt in der Tatsache begründet, dass der Ultrafiltrationsfluss in einem Zeitraum vom Start der sequentiellen Phase bis zum Abtransport urämischer Toxine, welcher üblicherweise unter zehn Minuten liegt, vollständig abgestellt sein kann. Nach Ende des Zeitraumes wird ein Dialysierflüssigkeitsfluss (Ultrafiltrationsfluss) eingestellt, der das im Dialysator angesättigte Dialysat zur Messeinrichtung befördert.

Entscheidend für die Messmethode unabhängig von der Art der Realisierung ist, dass die Messwertaufnahme möglichst schnell bzw. möglichst nah am Dialysator erfolgt. Die örtliche Nähe kann durch die Positionierung der Messeinrichtung im Dialysatkreislauf variiert werden. Die Geschwindigkeit der Messwertaufnahme wird im Wesentlichen durch die Höhe des Ultrafiltrationsflusses sowie das dialysatseitige Volumen erreicht.

Eine weitere Anwendung dieses erfindungsgemäßen Verfahrens ist auch die Bestimmung der Reinigungsleistung von Dialysatoren entweder zur Überprüfung der im Datenblatt angegebenen Werte oder zur Überprüfung der Reinigungsleistung des Dialysators bei Wiederverwendung. Das Vorgehen ist identisch mit den in den vorherigen Abschnitten skizzierten Punkten:

Der Absorptionskoeffizient des angesättigten Dialysats (Ultrafiltrates) ist ein Maß für die tatsächliche Konzentration urämischer Toxine im Blut dialysepflichtiger Patienten. Nach Abschluss der Messung kann nun durch das erneute Zuschalten des Dialysatflusses über den Dialysator mit der vorgesehenen Behandlungsmethode fortgefahren werden. Wird nun ein spezifischer Blutfluss eingestellt, so stellt sich die Reinigungsleistung des Dialysators entsprechend ein. Bedingt durch die Reinigungsleistung geht nur ein gewisser Teil urämischer Toxine in das Dialysat über und wird dort von der UV-Messeinrichtung erfasst. Nach Auswertung der zugehörigen Absorbanz kann nun die tatsächliche Reinigungsleistung des Dialysators bestimmt werden. Dazu müssen allerdings Blut- ($Q_{BF}$) und Dialysatfluss ($Q_{DF}$) berücksichtigt werden.

Stellt $A_{uF}$ die Absorbanz (proportional zur Konzentration) urämischer Toxine im reinen Ultrafiltrat und $A_{DF}$ die Absorbanz urämischer Toxine im verbrauchten Dialysat dar, so ergibt sich die Reinigungsleistung des Dialysators nach Umschalten auf die HD/HDF-Therapie wie folgt (2):

$$(2) \qquad K[\%] = \frac{A_{DF}}{A_{UF}} \cdot \frac{Q_{DF}}{Q_{BF}} \text{ bzw. für den aktuellen } Q_{BF}: K[ml/\min] = \frac{A_{DF}}{A_{UF}} \cdot Q_{DF}$$

Die Messung der Reinigungsleistung kann zu jedem beliebigen Zeitpunkt in der Behandlung durchgeführt werden. Voraussetzung ist allerdings, dass zuvor die absolute Konzentration urämischer Toxine im Blut des Dialysepatienten bestimmt wurdey.

Offenbart wird folglich ein Verfahren sowie eine Vorrichtung zur Bestimmung der Effektivität einer aktuell durchgeführten Nierenersatzbehandlung auf der Dialyseseite unter Verwendung einer Dialysemaschine, die in einem ersten Schritt in einem Hämodialyse- oder Hämodiaflitrationsprozess betrieben wird und in einem zweiten Schritt in einen sequentiellen Modus, in dem lediglich ein Fluss vom Blutkompartment (Flüssigkeitsentzug des Patienten) über die semipermeable Membran zum Dialysierflüssigkeitskompartment des Dialysators erzeugt wird, umgeschaltet wird, in welchem gemäß einem dritten Schritt ein dialyseseitig einem Dialysator nachgeschalteter Sensor zur Bestimmung oder Messung der Konzentration zumindest von urämischen Toxinen im angesättigten Dialysat (Ultrafiltrat) entsprechende Messsignale an eine Berechnungs- oder Bestimmungseinheit ausgibt, welche repräsentativ für die aktuelle Konzentration zumindest von urämischen Toxinen im Blut sind.

Im Nachfolgenden wird die Erfindung anhand eines bevorzugten Ausführungsbeispiels unter Bezugnahme auf die begleitenden Figuren näher beschrieben.

Fig. 1 zeigt den prinzipiellen Aufbau einer Dialysemaschine mit einer Messeinrichtung gemäß der vorliegenden Erfindung und

Fig. 2 zeigt beispielhaft den Messsignalverlauf im Fall erfindungsgemäß eingeleiteter sequentieller Phasen.

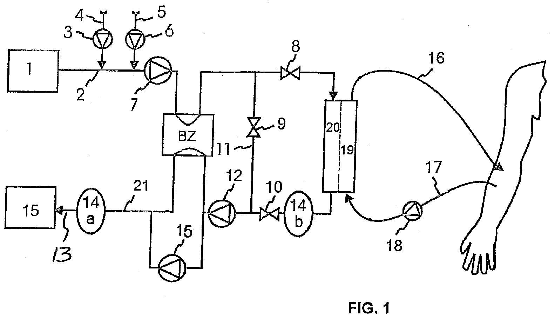

Gemäß der Fig. 1 hat die Dialysemaschine wenigstens eine Versorgungspumpe bzw. Flusspumpe-Dialysator-Eingang (FPE) 7, die eine Dialyseflüssigkeit über eine Zuführleitung einem Dialysator 19, 20 an dessen Eingangsseite zuführt. Die Dialyseflüssigkeit wird vorliegend vorbereitet, indem Wasser aus einer Wasseraufbereitung 1 über einen Wasserzufluss 2 der Zuführpumpe 7 zugeleitet wird, wobei dem Wasser durch eine Bikarbonat-Pumpe 3 ein Bikarbonat-Konzentrat 4 sowie durch eine Säure-Pumpe 6 ein Säure-Konzentrat 5 zugeführt wird. In die Zuführleitung ist zudem ein Bilanzierungssystem BZ zwischengeschaltet.

An einer Ausgangsseite des Dialysators 19, 20 ist eine Abflussleitung angeschlossen, die zu einer ersten Entsorgungspumpe oder Pumpeinrichtung am Dialysatorausgang 12 führt, welche verbrauchte Dialysierflüssigkeit (Dialysat) ebenfalls über das Bilanzierungssystem BZ einem Sammeltank bzw. Dialysatabfluss 15 zuführt. Parallel zum Bilanzierungssystem BZ ist an die erste Entsorgungspumpe 12 eine zweite Verstärkerpumpe 15 angeschlossen, die ebenfalls in den Sammeltank/Dialysatabfluss 15 fördern kann.

Es ist ein Umgehungskanal (Bypassleitung) 11 vorgesehen, der an die Zuführleitung und die Abflussleitung bzw. an den Ein- und Auslass des Dialysators 19, 20 unter Kurzschließen des Dialysators 19, 20 angeschlossen ist und in dem ein Öffnungs-/Schließventil 9 zwischengeschaltet ist. Des Weiteren ist unmittelbar vor dem Dialysatoreinlass stromab zur Anschlussstelle des Umgehungskanals 11 ein weiteres Öffnungs-/Schließventil 8 angeordnet.

An dem Dialysatorauslass ist unmittelbar daran eine Messeinrichtung 14b angeordnet, welche für die Messung der Konzentration an urämischen Toxinen angepasst ist. Dieser Messeinrichtung 14b unmittelbar nachgeschaltet ist ein letztes Öffnungs-/Schließventil 10, das stromauf zur Anschlussstelle des Umgehungskanals 11 angeordnet ist. Alternativ oder zusätzlich zu der Messeinrichtung 14b kann eine gleiche oder gleichartige Messeinrichtung 14a auch an der Abflussseite der Verstärkerpumpe 15 angeordnet sein.

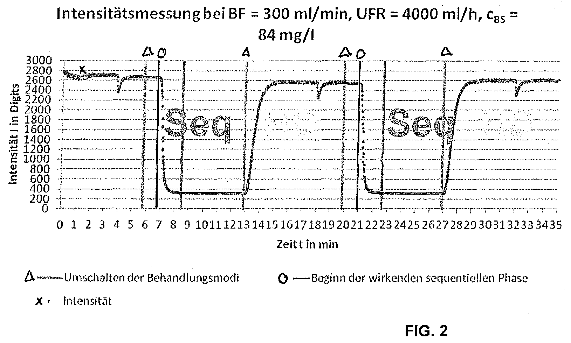

Die Funktion der Dialysemaschine mit vorstehendem konzeptionellem Aufbau lässt sich anhand der Fig. 2 wie Folgt umschreiben:

Während einer normalen Hämodialyse (entspricht den ungefähren Behandlungszeiträumen 0 - 6min, 13 - 20min, etc. gemäß der Fig. 2) sind die Ventile 8 und 10 geöffnet und das Ventil 9 geschlossen. Das frisch aufbereitete Wasser, welches durch Bikarbonat-Konzentrat 4 und das Säure-Konzentrat 5 in eine physiologisch gut verträgliche Dialysierlösung umgewandelt wird, durchfließt, angetrieben durch die Pumpen 7 und 12 zunächst das Bilanzierungssystem BZ, welches sicherstellt, dass die ein- und ausfließende Flüssigkeit in und aus dem Dialysator 19, 20 volumenmetrisch identisch sind. Nach Durchströmen des Bilanzierungssystems BZ wird die Dialysierlösung dann über das Ventil 8, den Dialysator 19, 20 und das Ventil 10 mittels der Pumpe 12 wieder in das Bilanzierungssystem BZ gepumpt.

Im Dialysator 19, 20 kommt es über eine semipermeable Membran (nicht näher gezeigt) mit Blut in Funktionskontakt, welches, angetrieben durch eine Blutpumpe 18 in einer Blutzuführleitung 17, den Dialysator 19, 20 auf dessen Blutseite passiert. Der Stoffaustausch erfolgt hier meistens mittels Diffusion.
Um eine Messung der Konzentration an urämischen Toxinen im Blut durchzuführen, wird eine sequentielle Phase gestartet (entspricht den ungefähren Behandlungszeiträumen 6 - 13min und 20 - 27min gemäß der Fig. 2). Hierfür wird das Ventil 8 geschlossen und das Ventil 9 geöffnet, während die Pumpen 7 und 12 unverändert weiterfördern. In diesem Fall fließt die Dialysierflüssigkeit nun nicht mehr über den Dialysator 19, 20 sondern über den Umgehungskanal 11. Die Verstärkerpumpe 15 hingegen fördert nunmehr zusätzlich (zur Erzeugung eines Unterdrucks auf der Dialyseseite im Dialysator) ausschließlich Flüssigkeit (Ultrafiltrat) aus dem Dialysator 19, 20, welche dem Blut entzogen wird. Die Fördermenge muss dabei entsprechend der Fördermenge der anderen Pumpen 7 und 12 zur Erzielung des angestrebten Druckgefälles im Dialysator angepasst werden.

Passiert die angesättigte Dialysierflüssigkeit (Ultrafiltrat gemäß vorstehender Definition) nun die Messeinrichtung 14b und/oder 14a, so kann die tatsächliche Menge urämischer Toxine im Blut des Patienten (entspricht der Konzentration urämischer Toxine im reinen Ultrafiltrat) dialyseseitig (nicht blutseitig) gemessen werden. Hier sei darauf hingewiesen, dass im Fall der Messeinrichtung 14a das Ultrafiltrat bereits wieder mit der noch frischen Dialysierflüssigkeit aus dem Umgehungskanal 11 vermischt vorliegt, da sich diese Messeinrichtung 14a stromab zum Umgehungskanal 11 befindet.

Nach Abschluss der Messung öffnet das Ventil 8 wieder, während das Ventil 9 schließt, sodass der ursprüngliche Dialysatfluss den Dialysator wieder passieren kann.

Aus der Fig. 2 ist zu entnehmen, dass nach dem Umschalten von z.B. einer gewöhnlichen Dialyse-Therapie (Hämodialyse- (HD) oder Hämodiaflitrationsprozess (HDF)) in eine sequentielle Phase (bzw. Hämofiltrationsprozess (HF)) gemäß vorstehender Definition die Intensität der UF-Messeinrichtung auf einen konstanten Wert absinkt, welcher die Konzentration urämischer Toxine im Blut wiederspiegelt. Der erreichte Intensitätswert stellt dabei nach der Gleichung (1) die Konzentration dar. Natürlich gilt das Verfahren formell auch für HF-Therapien, allerdings ist die Anwendung dort trivial, da kein Umschalten erfolgen muss, da die Therapie ausschließlich in sequentiellem Modus betrieben wird. Außerdem kommt diese Form der Therapie sehr selten zum Einsatz.
Die mit dem erfindungsgemäßen Verfahren gemessene Absorbanz $A_{UF}$ kann ausschließlich dazu verwendet werden, um mittels der Gleichung (2) die tatsächliche Reinigungsleistung des Dialysators während einer Hämodialysetherapie zu bestimmen.

Des Weiteren ist es möglich und auch aus wirtschaftlichen Gründen empfehlenswert, die sequentielle Phase bereits zu beenden, bevor der Messendwert erreicht wird. Die Idee hierbei besteht darin, das Messverfahren wesentlich zu beschleunigen, indem z.B. lediglich eine kurzzeitige, vorzugsweise ein-minütige sequentielle Phase gefahren wird, bevor wieder auf die HD-Therapie zurückgeschaltet wird, die per se nicht lange genug ist, bis die Flüssigkeit den Senor erreicht. Da sich die angesättigte Flüssigkeit (Ultrafiltratsprobe) nämlich bereits im Leitungssystem der Dialysemaschine befindet, braucht somit nicht mehr abgewartet zu werden, bis dieses auch den Sensor erreicht hat. Dies wird auch nach dem Beenden der sequentiellen Phase zwangsläufig durch Zuschalten des Dialysatflusses durch den Dialysator eintreten. In diesem Fall muss nur sichergestellt sein, dass die Zeitdauer der sequentiellen Phase ausreicht, um ein genügend großes Volumen (Flüssigkeitsstrecke in der Leitung) blutseitiger Flüssigkeit in das dialysatseitige Leitungssystem zu fördern. Es hat sich hierbei ergeben, dass - abhängig von der Pumpendrehzahl im Bereich von 1 - 100ml/min - bereits nach wenigen Sekunden bis Minuten das geförderte Flüssigkeitsvolumen ausreichend ist.

Bezugszeichenliste

1.   Wasseraufbereitung
2.   Wasserzufluss
3.   Bikarbonat-Pumpe
4.   Bikarbonat-Konzentrat
5.   Säure-Konzentrat
6.   Säure-Pumpe
7.   Flusspumpe-Dialysator-Eingang (FPE)
8.   Ventil Dialysator Eingang (VDE)

9. Bypass-Ventil (VBP)
10. Ventil Dialysator Ausgang (VDA)
11. Bypass-Leitung
12. Pumpeinrichtung am Dialysatausgang bzw. Flusspumpe-Dialysator-Ausgang (FPA)
13. Dialysatabflussleitung
14a. UV-Messeinrichtung (Position 1)
14b. UV-Messeinrichtung (Position 2)
15. Dialysatabfluss
16. venöses Schlauchsystem
17. arterielles Schlauchsystem
18. arterielle Blutpumpe (BPA)
19. Blutseite (BS) im Dialysator
20. Dialysatseite (DS) im Dialysator

**Patentansprüche**

1. Dialysemaschine mit einer Messeinrichtung zur Messung und/oder Bestimmung der Konzentration zumindest von urämischen Toxinen in Patientenblut aufweisend:- einen Dialysator (19, 20), der auf seiner Eingangsseite von wenigstens einer Versorgungspumpe (7) mit einer frischen Dialysierflüssigkeit anströmbar ist, - wenigstens eine Entsorgungspumpe (12, 15), welche an die Ausgangsseite des Dialysators (19, 20) angeschlossen ist, um das zumindest mit urämischen Toxinen kontaminierte Dialysat aus dem Dialysator (19, 20) zu fördern, - eine Ventileinrichtung (8, 9, 10), welche in einem Hämodialyse- oder Hämodiaflitrationsmodus der Maschine eine Fluidverbindung der wenigstens einen Versorgungspumpe (7) zum Dialysator (19, 20) zulässt und in einem sequentiellen Modus, in dem lediglich ein Fluss vom Blutkompartment über die semipermeable Membran zum Dialysierflüssigkeitskompartment des Dialysators erzeugt wird oder in einem Hämofiltrationsmodus der Maschine diese Fluidverbindung unterbricht und stattdessen einen Umgehungskanal (11) zum Dialysator (19, 20) öffnet und wenigstens eine Sensoreinrichtung (14a, 14b), welche dem Dialysator (19, 20) nachgeschaltet ist und dafür ausgebildet ist, im sequentiellen Modus oder im Hämofiltrationsmodus der Maschine Messsignale an eine Berechnungs- und/oder Bestimmungseinheit zu senden, welche die Konzentration zumindest von urämischen Toxinen im angesättigten Dialysat oder Ultrafiltrat angeben **dadurch gekennzeichnet, dass** die Ventileinrichtung (8, 9, 10) der Dialysemaschine so angepasst ist, dass die Dialysemaschine zeitlich vor der Beendigung des Mess-Betriebsschritts bereits in den Hämodialyse- oder Hämodiafiltrationsprozess zurückgeschaltet wird und wobei die Ventileinrichtung (8, 9, 10) weiterhin so angepasst ist, dass der sequentielle Modus oder Hämofiltrationsprozess nur so lange durch die Ventileinrichtung geschaltet ist dass ein für die Messung ausreichendes Volumen blutseitiger Flüssigkeit in ein dialysatseitiges Leitungssystem gefördert ist, das zu dem Sensor führt.

2. Dialysemaschine nach Anspruch 1, **dadurch gekennzeichnet, dass** die Ventileinrichtung (8, 9, 10), die Messeinrichtung und die Berechnungs- oder Bestimmungseinrichtung dazu ausgelegt sind, dass der Umschalt-Betriebsschritt sowie der nachfolgende Mess-Betriebsschritt mindestens zu Beginn, und vorzugsweise auch während und am Ende einer Dialysebehandlung ausgeführt werden.

3. Dialysemaschine nach einem der vorstehenden Ansprüche, **dadurch gekennzeichnet, dass** Messeinrichtung (14a, 14b) UV Sensoren sind und zur Messung der Konzentration zumindest von urämischen Toxinen die UV-Spektrometrie eingesetzt ist.

4. Dialysemaschine nach einem der vorstehenden Ansprüche, **dadurch gekennzeichnet, dass** die Messeinrichtung (14a, 14b) und die Berechnungs- oder Bestimmungseinrichtung dazu ausgelegt sind, dass die Absorbanz als Maß für die Konzentration zumindest von urämischen Toxinen bestimmt wird.

5. Dialysemaschine nach einem der vorstehenden Ansprüche, **dadurch gekennzeichnet, dass** die Ventileinrichtung (8, 9, 10) so ausgelegt ist, dass nach Beendigung des Mess-Betriebsschritts die Dialysemaschine in den Hämodialyse- oder Hämodiafiltrationsprozess zurückgeschaltet wird.

6. Dialysemaschine nach Anspruch 1, **dadurch gekennzeichnet, dass** der Umgehungskanal (11) in eine Verbindungsleitung zwischen dem Dialysator (19, 20) und der Entsorgungspumpe (12) einmündet.

7. Dialysemaschine nach Anspruch 6, **dadurch gekennzeichnet, dass** die Sensoreinrichtung (14b) an die Verbindungsleitung zwischen dem Dialysator (19, 20) und der Entsorgungspumpe (12) angeschlossen ist.

8. Dialysemaschine nach Anspruch 7, **dadurch gekennzeichnet, dass** die Sensoreinrichtung (14a) der Entsorgungspumpe (12) nachgeordnet ist.

**Claims**

1. A dialysis machine comprising a measuring device for measuring and/or determining the concentration of at least uremic toxins in a patient's blood, comprising: a dialyser (19, 20) against the input side of which, fresh dialysis fluid can flow from at least one supply pump (7), at least one discharge pump (12, 15) connected to the output side of the dialyser (19, 20) so as to convey the dialysate (19, 20) contaminated at least with uremic toxins from the dialyser (19, 20), a valve means (8, 9, 10) which permits fluid communication of the at least one supply pump (7) to the dialyser (19, 20) in a hemodialysis or hemodiafiltration mode of the dialysis machine and in a sequential mode in which merely a flow from the blood compartment via the semipermeable membrane to the dialysis fluid compartment of the dialyser is generated or in a hemofiltration mode of the machine disconnects said fluid communication and instead opens a bypass channel (11) to the dialyser (19, 20) and at least one sensor means (14a, 14b) connected downstream of the dialyser (19, 20) and being configured to send, in the sequential mode or in the hemofiltration mode of the dialysis machine, measuring signals indicative of the concentration at least of uremic toxins in the saturated dialysate or ultra-filtrate to a calculation and/or determination unit,
**characterized in that**
the valve means (8, 9, 10) of the dialysis machine is configured such that the dialysis machine is already changed back to the hemodialysis or hemodiafiltration process temporally before completion of the measuring operating step and wherein the valve means (8, 9, 10) is furthermore configured such that the sequential process or the hemofiltration mode is only switched through the valve means for as long as a volume of blood-side fluid sufficient for measuring is conveyed into a dialysis-side tubing system leading to the sensor

2. The dialysis machine according to claim 1, **characterized in that** the valve means (8, 9, 10), the measuring device, and the calculation and/or determination unit are configured such that the change-over operating step as well as the following measuring operating step are carried out at least at the beginning and preferably also during and at the end of a dialysis treatment.

3. The dialysis machine according to one of the preceding claims, **characterized in that** UV sensors are the measuring device (14a, 14b) and UV spectrometry is employed for measuring the concentration of at least uremic toxins.

4. The dialysis machine according to one of the preceding claims, **characterized in that** the measuring device (14a, 14b) and the calculation and/or determination unit are configured such that the absorbance is determined as a measure for the concentration of at least uremic toxins.

5. The dialysis machine according to one of the preceding claims, **characterized in that** the valve means (8, 9, 10) is configured such that after completion of the measuring operating step, the dialysis machine is changed back to the hemodialysis or hemodiafiltration process.

6. The dialysis machine according to claim 1, **characterized in that** the bypass channel (11) opens into a connecting line between the dialyser (19, 20) and the discharge pump (12).

7. The dialysis machine according to claim 6, **characterized in that** the sensor means (14b) is connected to the connecting line between the dialyser (19, 20) and the discharge pump (12).

8. The dialysis machine according to claim 7, **characterized in that** the sensor means (14a) is connected downstream of the discharge pump (12).

**Revendications**

1. Machine de dialyse comprenant un dispositif de mesure servant à mesurer et/ou à déterminer la concentration au moins de toxines urémiques présentes dans le sang d'un patient, présentant : - un dialyseur (19, 20), qui peut être parcouru, sur son côté d'entrée depuis au moins une pompe d'alimentation (7), par un liquide de dialyse frais, - au moins une pompe d'évacuation (12, 15), qui est raccordée au côté de sortie du dialyseur (19, 20) afin de refouler hors du dialyseur (19, 20) le dialysat au moins au nombre de un contaminé par des toxines urémiques, - un dispositif de vanne (8, 9, 10), qui permet une liaison fluidique de la pompe d'alimentation (7) au moins au nombre de une avec le dialyseur (19, 20) dans un mode d'hémodialyse ou d'hémodiafiltration de la machine et qui interrompt ladite liaison fluidique dans un mode séquentiel, dans lequel uniquement un flux est produit depuis le compartiment de sang, en passant par de la membrane semi-perméable, vers le compartiment de liquide de dialyse du dialyseur ou dans un mode d'hémofiltration de la machine et ouvre, en lieu et place, un canal de contournement (11) menant au dialyseur (19, 20), et - au moins un dispositif de capteur (14a, 14b), qui est branché en aval du dialyseur (19, 20) et qui est réalisé afin d'envoyer dans le mode séquentiel ou dans le mode d'hémofiltration de la machine des signaux de mesure à une unité de calcul et/ou de détermination, lesquels indiquent la concentration au moins de toxines urémiques présentes dans le dialysat ou l'ultrafiltrat non saturé, **caractérisée en ce que** le dispositif de vanne (8, 9, 10) est adapté à la machine de dialyse de telle sorte que la machine de dialyse est éteinte chronologiquement avant la fin de l'étape opérationnelle de mesure déjà dans le processus d'hémodialyse ou d'hémofiltration, le dispositif de vanne (8, 9, 10) étant par ailleurs adapté de telle sorte que le mode séquentiel ou le processus d'hémofiltration est activé par le dispositif de vanne seulement tant qu'un volume suffisant pour la mesure de liquide côté sang est refoulé dans un système de conduit côté dialysat, lequel mène au capteur.

2. Machine de dialyse selon la revendication 1, **caractérisée en ce que** le dispositif de vanne (8, 9, 10), le dispositif de mesure et le dispositif de calcul ou de détermination sont configurés de sorte que l'étape opérationnelle de commutation ainsi que l'étape opérationnelle de mesure qui suit sont exécutées au moins au début, et de préférence également pendant et à la fin du traitement par dialyse.

3. Machine de dialyse selon l'une quelconque des revendications précédentes, **caractérisée en ce que** les dispositifs de mesure (14a, 14b) sont des capteurs UV, et **en ce que** la spectrométrie par UV est utilisée aux fins de la mesure de la concentration au moins de toxines urémiques.

4. Machine de dialyse selon l'une quelconque des revendications précédentes, **caractérisée en ce que** le dispositif de mesure (14a, 14b) et le dispositif de calcul ou de détermination sont configurés de manière à ce que l'absorbance soit déterminée en tant que mesure de la concentration au moins de toxines urémiques.

5. Machine de dialyse selon l'une quelconque des revendications précédentes, **caractérisée en ce que** le dispositif de vanne (8, 9, 10) est configuré de telle sorte qu'à l'issue de l'étape opérationnelle de mesure, la machine de dialyse est commutée à nouveau dans le processus d'hémodialyse ou d'hémodiafiltration.

6. Machine de dialyse selon la revendication 1, **caractérisée en ce que** le canal de contournement (11) débouche dans un conduit de liaison entre le dialyseur (19, 20) et la pompe d'évacuation (12).

7. Machine de dialyse selon la revendication 6, **caractérisée en ce que** le dispositif de capteur (14b) est raccordé au conduit de liaison entre le dialyseur (19, 20) et la pompe d'évacuation (12).

8. Machine de dialyse selon la revendication 7, **caractérisée en ce que** le dispositif de capteur (14a) est disposé en aval de la pompe d'évacuation (12).

FIG. 1

Intensitätsmessung bei BF = 300 ml/min, UFR = 4000 ml/h, $c_{BS}$ = 84 mg/l

Δ▬▬Umschalten der Behandlungsmodi     O ——Beginn der wirkenden sequentiellen Phase

X · Intensität

**FIG. 2**

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 0986410 A
- EP 2005982 B1
- US 5849179 A